# EUROPEAN PATENT APPLICATION

(11) **EP 4 431 085 A1**
(43) Date of publication of application: **18.09.2024**
(21) Application number: 23197805.7
(22) Date of filing: 15.09.2023
(51) Int. Cl.: A61K 9/127, A61K 9/51, A61K 31/7105, A61K 31/711, A61K 47/14, C12N 15/88, A61K 48/00

(54) **LIPID COMPOSITION AND SUBSTANCE DELIVERY METHOD**

(30) Priority: 17.03.2023 JP 2023043358
(71) Applicant: KABUSHIKI KAISHA TOSHIBA, Minato-ku Tokyo 105-0023 (JP)
(72) Inventor: NOZAKI, Emi, Tokyo (JP); AKAHOSHI, Eiichi, Tokyo (JP); ISHIHARA, Mitsuko, Tokyo (JP)
(74) Representative: Marks & Clerk LLP

(57) **Abstract**

According to an arrangement, a lipid composition is for delivering an objective substance (3) to a target cell. The composition is contacted with the target cell under an environment of 37°C or higher. The composition includes a substance delivery carrier having lipid particle (1) and an objective substance (3) encapsulated in the lipid particle (1). The lipid particle (1) constitutes a liposome and includes FFT-10 and/or FFT-20 as constituents thereof.

## Description

### FIELD

The present disclosure relates to a lipid composition and a substance delivery method.

### BACKGROUND

For example, in the case of producing a target cell by genomic recombination, such as a case of CAR-T cell, more efficiently delivering an objective molecule to a target cell is desirable under circumstances where multiple types of cells exist.

### BRIEF DESCRIPTION OF THE DRAWINGS

FIG. 1 is a cross sectional view showing an example of a substance delivery carrier of the first arrangement.
FIG. 2 is a flow chart showing an example of the second and sixth arrangements.
FIG. 3 is a flow chart showing an example of the third and seventh arrangements.
FIG. 4 is a flow chart showing an example of the fourth and eighth arrangements.
FIG. 5 is a schematic view showing an example of the fourth arrangement.
FIG. 6 is a flowchart showing an example of fifth and ninth arrangements.
FIG. 7 is a schematic view of a region warming mechanism used in an example according to the ninth arrangement.
FIG. 8 is a graph showing the results of Experiment 1.
FIG. 9 is a graph showing results of Experiment 2.
FIG. 10 is an image showing the result of Experiment 4.

### DETAILED DESCRIPTION

According to one arrangement, a lipid composition is for delivering an objective substance to a target cell. The composition is contacted with the target cell under an environment of 37°C or higher. The composition includes a substance delivery carrier having lipid particle and an objective substance encapsulated in the lipid particle. The lipid particle constitutes a liposome, and includes FFT-10 and/or FFT-20 as constituents thereof.

According to an arrangement, for example, one of purposes is to provide a technology for highly efficiently delivering an objective substance to a target cell.

### (First Arrangement)

The lipid composition of the first arrangement is for delivering an objective substance. The composition is characterized by being contacted with target cells under an environment of 37°C or higher. The composition includes a substance delivery carrier a containing lipid particle and an objective substance encapsulated within the lipid particle. The lipid particle constitutes a liposome, and includes FFT-10 and/or FFT-20 as a constituent component thereof.

For example, such a lipid composition may include a substance delivery carrier comprising a lipid particle and an objective substance encapsulated within the lipid particle, and the lipid particle may include, as constituents thereof, a first lipid (FFT-10) of formula (I) and/or a second lipid (FFT-20) of formula (II) ; 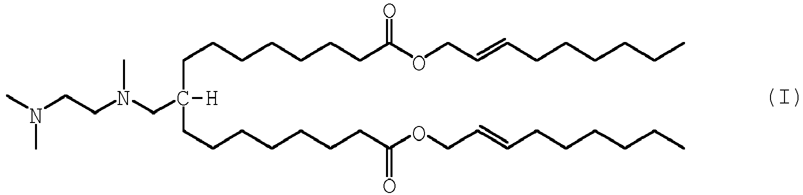 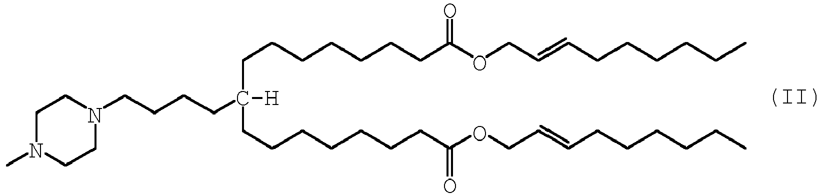 and also, the lipid composition is for delivering an objective substance to a target cell, and characterized by being brought into contact with the target cell under an environment of 37°C or higher.

The target cell may be a cell to which the lipid composition according to the arrangement is delivered. For example, the cells may be any non-normal cells, abnormal cells, cells affected by a disease, cells pull out of a normal range, or the like. Specifically, it may be, for example, a cancer cell and a tumor cell. Examples of cancer cells and tumor cells include, but are not limited to, breast cancer, colon cancer, lung cancer, stomach cancer, pancreatic cancer, cervical cancer, uterine cancer, ovarian cancer, sarcoma, prostate cancer, bile duct cancer, urinary bladder cancer, esophageal cancer, liver cancer, brain tumor, kidney cancer, T cell tumor cells, and the like. For example, a T cell tumor is a disease caused by malignant tumorigenesis of T cells (T lymphocytes). The T cell tumor cells include malignant tumor T cells, for example, leukemia cells, lymphoma cells, and the like derived from T cells.

Examples of the objective substance may be a therapeutic agent and/or a diagnostic agent, an index substance for specifying the property of the target cell, a substance for obtaining information useful for diagnosis and judgment of treatment, a substance that kills the target cell, a substance for recombining the genome of the target cell, and the like. For example, the substances may be natural products, compounds, extracts, nucleic acids, peptides, proteins, and the like. For example, the nucleic acid may be a nucleic acid fragment, a nucleic acid construct, DNA, RNA, and the like.

A composition according to an arrangement is for delivering an objective substance to a target cell under an environment of 37°C or higher. As a physical property of the substance delivery carrier, when the substance delivery carrier comes into contact with the target cell at the higher temperature than normal culture temperature, one feature of achieving introduction of the objective substance encapsulated in the lipid particle into the target cell with higher efficiency is exhibited. In addition, for example, in a case where the objective substance is a substance intended to be delivered to the cell nucleus, the objective substance can achieve introduction into the nucleus of the target cell with high efficiency.

The environment of 37°C or higher may be an environment provided under a temperature condition higher than the temperature of normal culture conditions as described above, and such a temperature may be, for example, 37°C or higher, or higher than 37°C, for example, 40°C to 46°C, or 40°C to 42°C. The contact time may be from 5 minutes to 20 minutes, such as from 7 minutes to 18 minutes, or from 8 minutes to 17 minutes, and the like. As an appropriate contact condition, for example, a combination of temperature and time may be appropriately selected according to the cell type. The temperature may be achieved by adding the lipid composition to the target cell and then warming, for example, warming after the target cell and the delivery carrier come into contact with each other, adding the lipid composition to the target cell while warming, or the like. Preferably, the lipid composition is warmed after being added to the target cell. Here, for example, warming at 42°C or higher, or warming for 20 minutes or longer tends to accelerate cell death, therefore, depending on the combination of temperature and time, a condition lower than that may be preferable.

A composition according to an arrangement is a for delivering an objective substance, a therapeutic substance, and/or a substance, a synthetic compound, a nucleic acid substance, or the like for obtaining information regarding treatment or diagnosis to a target cell, for example, a non-normal cell such as a cancer cell, or to the genome of the target cell. The center of the activity of this composition is a substance delivery carrier including a lipid particle and an objective substance encapsulated within the lipid particle. The objective substance is delivered to the target cell, for example, into the target cell, into the nucleus of the target cell, and/or the genome of the target cell by the configuration of the lipid particle and the use condition of being brought into contact with the target cell under an environment of 37°C or higher. Here, "delivering an objective substance to a target cell" comprehensively includes, for example, delivering the objective substance to the entire target cell or a local site of the target cell such as in the target cell or cell, in the nucleus or nucleus of the target cell, or in the genome or genome of the target cell. Specifically, for example, it can depend on the type and nature of the objective substance to be delivered, and the purpose of delivery.

### · Lipid particle

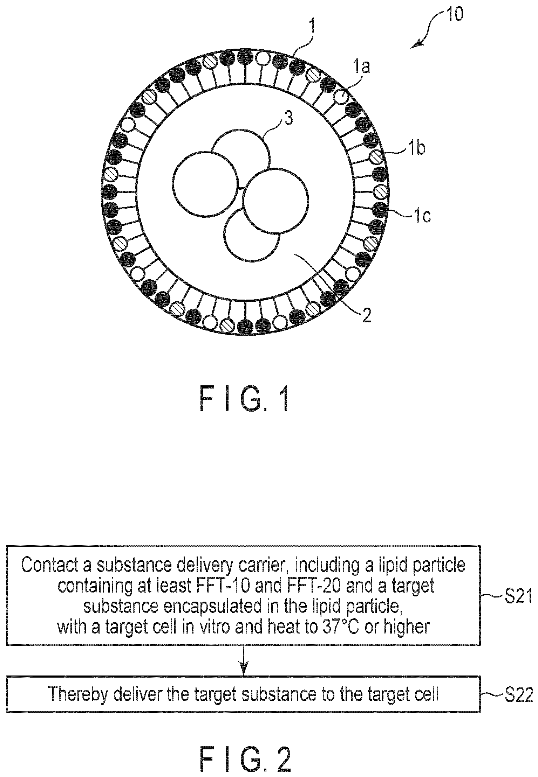

As shown in FIG. 1, the lipid particle 1 is a substantially spherical hollow body, and can contain the objective substance 3 in the lumen 2 at the center thereof.

The objective substance 3 is a substance desired to be delivered into a target cell. The objective substance 3 may be any substance as long as it can be encapsulated in the lipid particle 1, but as described above, can be, for example, a nucleic acid, a protein, a peptide, another organic compound, an inorganic compound, a therapeutic agent, a diagnostic agent, or the like.

The lipid particle 1 can be composed of, for example, a lipid membrane formed by arranging a plurality of lipid molecules as a material thereof in a non-covalent manner. The lipid particle 1 may include at least the first lipid 1a and/or the second lipid 1b as a constituent component thereof. However, it is particularly desirable for obtaining an action that the lipid particle 1 contains at least the first lipid 1a and the second lipid 1b as constituent components thereof. The first lipid 1a is a lipid compound (FFT-10) having the following formula (I), and the second lipid 1b is a lipid compound (FFT-20) having the following formula (II).

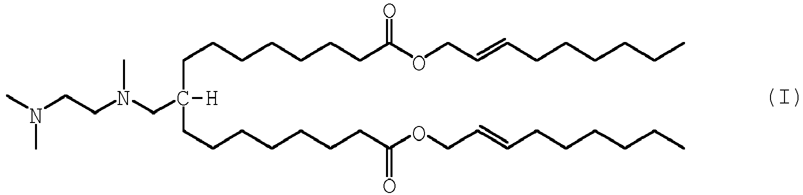

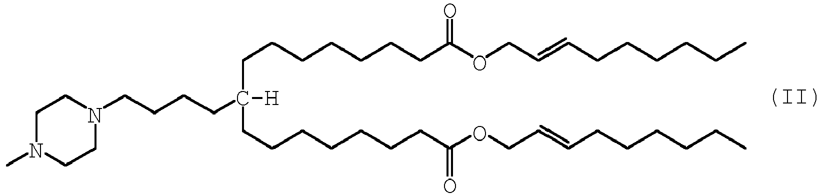

The lipid particle 1 may contain an additional lipid in addition to the first lipid 1a and the second lipid 1b. In the composition of the lipid molecular material constituting the lipid particle 1, a fraction composed of the first lipid 1a and the second lipid 1b is hereinafter referred to as a "first fraction". In addition, a fraction composed of a lipid molecular material other than the first lipid 1a and the second lipid 1b is hereinafter referred to as a "second fraction". The lipids contained in the second fraction are hereinafter also collectively referred to as "third lipid 1c".

The terms first fraction and second fraction represent the composition of the constituent components of the lipid particle 1, and do not indicate the physical location of the lipids contained therein. For example, the constituent components of the first fraction and the second fraction do not need to be combined into one group in the lipid particle 1, and the lipid contained in the first fraction and the lipid contained in the second fraction can be mixed and present. The mixing ratio of the first fraction with respect to the entire lipid material constituting the lipid particle 1 may be 5% or more, 100 or more, 15% or more, for example, 10% to 80%, 15% to 60%, or the like.

In other words, regarding the total content of the FFT-10 and the FFT-20, the mixing proportion in the lipid particle may be 5% or more, 8% or more, 100 or more, 15% or more, for example, 10% to 80%, or 15% to 60%, 15% to 50%, or the like. The maximum content of FFT-10 and FFT-20 in the lipid particle may be, for example, an amount in which the lipid particle can be in a form liposome. The mixing ratio of the second lipid 1b in the first fraction may be 0% or more to 100%, and may be, for example, 15% to 75, 20% to 60%, 24% to 50%, or the like. Similarly, the mixing ratio of the first lipid 1a in the first fraction may be 0% or more to 100%, and may be, for example, 15% to 75, 20% to 60%, 24% to 50%, or the like.

Depending on the mixing ratio of the first lipid 1a and the second lipid 1b in the first fraction, the particle size of the lipid particle 1 and the permeability into cells may be changed. For example, the larger the second lipid 1b is, the larger the particle size of the lipid particle 1 can be. The average particle size of the lipid particle 1 can be changed according to the application. For example, the size may be adjusted to about 50 nm to about 300 nm. For example, when used in vivo, the size may be preferably about 70 nm to about 100 nm.

The lipid particle 1 can be taken up into the cell, for example, by endocytosis by being brought into contact with a target cell at 37°C or higher. The objective substance 3 can be released into the cell. Then, it can be delivered to the genome according to the objective substance 3. By a simple procedure of encapsulating the objective substance 3 in the lipid particle 1 and bringing it into contact with the target cell (for example, administering, adding, mixing, etc.), the objective substance 3 can be efficiently introduced into the target cell without using, for example, an antibody or a receptor as in the related art. Thus, the lipid particle 1 can be used in various applications in which it is required to selectively or specifically deliver the objective substance 3 to the target cell.

The type of the third lipid 1c contained in the second fraction of the lipid particles 1 is not limited, and for example, the second fraction contains a base lipid. As the base lipid, for example, a lipid which is a main component of a biological membrane can be used. The base lipid is a phospholipid or a sphingolipid, such as diacylphosphatidylcholine, diacylphosphatidylethanolamine, ceramide, sphingomyelin, dihydrosphingomyelin, kephalin, or cerebroside, or a combination thereof, and the like.

For example, as a base lipid,
1,2-dioleoyl-sn-glycero-3-phosphoethanolamine (DOPE),
1,2-stearoyl-sn-glycero-3-phosphoethanolamine (DSPE),
1,2 dipalmitoyl-sn-glycero-3-phosphatidylcholine (DPPC),
1-palmitoyl-2-oleoyl-sn-glycero-3-phosphatidylcholine (POPC),
1,2-di-O-octadecyl-3-trimethylammoniumpropane (DOTMA),
1,2-dioleoyl-3-dimethylammoniumpropane (DODAP),
1,2 dimyristoyl-3-dimethylammonium propane (14: 0 DAP),
1,2 dipalmitoyl-3-dimethylammonium propane (16: 0 DAP),
1,2 distearoyl-3-dimethylammonium propane (18: 0 DAP),
N-(4-carboxybenzyl)-N,N-dimethyl-2,3-bis(oleoyloxy)propane (DOBAQ),
1,2-dioleoyl-3-trimethylammoniumpropane (DOTAP),
1,2-dioleoyl-sn-glycero-3-phosphochlorin (DOPC),
1,2-dilinoleoyl-sn-glycero-3-phosphochlorin (DLPC),
1,2-dioleoyl-sn-glycero-3-phospho-L-serine (DOPS), or
cholesterol,
alternatively, a combination of any of these, or the like, are preferably used.

As the base lipid, it is particularly preferable to use a lipid of a cationic lipid or a neutral lipid, and the acid dissociation constant of the lipid particle 1 can be adjusted by the content thereof. DOTAP is preferably used as the cationic lipid, and DOPE is preferably used as the neutral lipid.

It is also preferred that the second fraction comprises lipids which prevent the aggregation of the lipid particles 1. For example, the lipid that prevents aggregation preferably further includes a PEG-modified lipid, for example, polyethylene glycol (PEG) dimyristoyl glycerol (DMG-PEG), a polyamide oligomer (US 6,320,017) derived from an omega-amino(oligoethylene glycol)alkanoic acid monomer, monosialoganglioside, or the like.

The second fraction may further contain lipids such as a relatively less toxic lipid for modulating toxicity; a lipid having a functional group that binds a ligand to the lipid particle 1; a lipid for suppressing leakage of an inclusion such as sterol, for example, cholesterol; and the like. In particular, it is preferable to contain cholesterol.

The type and composition of the lipid used in the second fraction are appropriately selected in consideration of the acid dissociation constant (pKa) of the target lipid particle 1 or the particle diameter of the lipid particle 1, the type of the objective substance 3, stability in cells, or the like.

For example, when the second fraction contains DOPE, DOTAP, cholesterol, and DMG-PEG, the delivery efficiency of the objective substance 3 is particularly excellent, which is preferable.

In addition to the objective substance 3, additional components may be encapsulated in the lipid particle 1 as necessary. The additional component is, for example, a pH adjusting agent, an osmotic pressure adjusting agent, a gene activator or another therapeutic agent for T cell tumor cells, another diagnostic agent, or the like. The pH adjusting agent is, for example, an organic acid such as citric acid, and a salt thereof, and the like. The osmotic pressure adjusting agent is a sugar, an amino acid, or the like. The gene activator will be described later.

The lipid particle 1 encapsulating the objective substance 3 and other substances as necessary can be produced, for example, using a known method used when a small molecule is enclosed in a lipid particle, for example, a Bangam method, an organic solvent extraction method, a surfactant removal method, a freeze-thaw method, or the like. For example, a lipid mixture obtained by including the material of the lipid particle 1 in an organic solvent such as an alcohol or the like at a desired ratio and an aqueous buffer containing a component to be included such as the objective substance 3 and the like are prepared, and the aqueous buffer is added to the lipid mixture. The obtained mixture is stirred and suspended to form the lipid particles 1 encapsulating the objective substance 3 and the like.

The mixing ratio of the constituent components of the lipid particle 1 can be easily adjusted by changing the mixing ratio of each material in the lipid mixture. For example, the mixing ratio of the constituent components of the lipid particle 1 may be substantially the same as the mixing ratio of each material in the lipid mixture. In addition, the amount ratio of the substances contained in the lipid particles 1 can be easily adjusted by changing the amount ratio of both in the aqueous buffer solution.

Hereinafter, the lipid particle 1 encapsulating the objective substance 3 is also referred to as a "substance delivery carrier" or a "delivery carrier". In FIG. 1, it is shown as a substance delivery carrier 10.

### · Composition

The substance delivery carrier may be provided as a liquid composition in a suitable carrier. The carrier is, for example, water, saline such as physiological saline, glycine aqueous solution, buffer solution, or the like. Alternatively, the substance delivery carrier may be provided as a dry, powdered composition. The powdery composition can be used by the user adding a suitable liquid such as the carrier.

In addition to the substance delivery carrier, the composition may further comprise a substance that improves storage stability. The substance that improves the storage stability may be, not limited, glycoproteins such as albumin, lipoprotein, apolipoprotein, globulin, and the like: pH adjusting agents, buffering agents, tonicity adjusting agents, and the like; agents which are pharmaceutically acceptable and bring the composition closer to physiological conditions, such as sodium acetate, sodium lactate, sodium chloride, potassium chloride, calcium chloride, and the like; fat affinity free radical quenchers such as α-tocopherol, which suppress damage by free radicals; lipid protecting agents such as water-soluble chelators such as ferrioxamine for suppressing lipid peroxidation damage and improving storage stability, and the like.

When the substance delivery carrier is used for administration to a living body, it is preferred that the composition has a pharmaceutically acceptable composition and has been sterilized in a known manner.

The production of the lipid particles 1 is carried out using a lipid mixture. The lipid mixture includes at least a first lipid 1a and a second lipid 1b. The lipid mixture contains the first lipid 1a and the second lipid 1b in any desired mixing ratio described above, and may also contain the second fraction of lipids. The lipid mixture may be provided as a kit for producing the lipid particle 1 together with the desired objective substance 3.

Lipid compositions or substance delivery carriers according to arrangements can be used to perform a variety of methods, depending on the target cell or substance used.

### (Second Arrangement)

According to a second arrangement, a method for delivering an objective substance to a target cell is provided. As shown in FIG. 2, the method includes contacting a substance delivery carrier containing a lipid particle containing at least FFT-10 and/or FFT-20 and an objective substance encapsulated in the lipid particle with a target cell in vitro and warming the carrier to 37°C or higher (S21), thereby delivering the objective substance to the target cell (S22). Note that FIG. 2 illustrates an example including both the FFT-10 and the FFT-20 as a preferable example.

The contact between the target cell and the substance delivery carrier is performed by adding, for example, the substance delivery carrier in the form of a lipid composition to the target cell contained in a culture vessel such as a petri dish, a culture dish or a culture tube, a culture tank, or any reaction vessel. Warming at 37°C or higher can be performed, for example, by maintaining in an incubator or heating using a warming device such as another heater or a warming system. The warming conditions are as described above. For example, the warming at 37°C or higher may be at 40 to 42°C.

### (Third Arrangement)

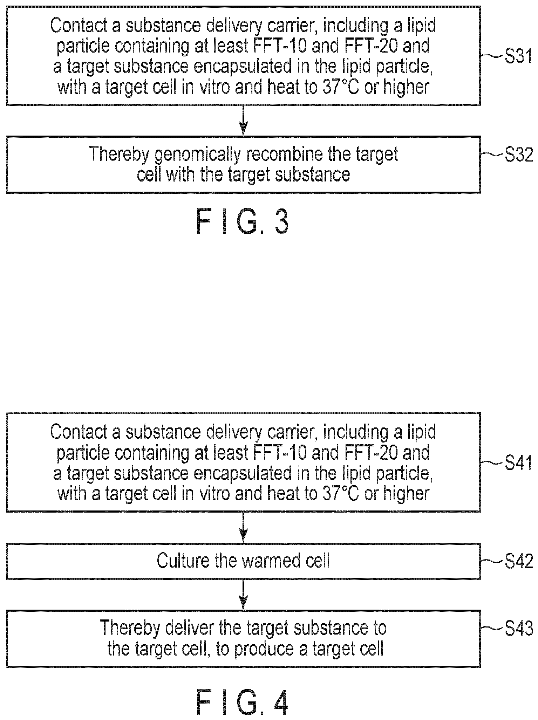

According to a third arrangement, there is provided a method for genomically recombining a target cell with an objective substance. As shown in FIG. 3, the method includes contacting a substance delivery carrier including a lipid particle containing at least FFT-10 and/or FFT-20 and an objective substance encapsulated in the lipid particle with a target cell in vitro and warming the carrier to 37°C or higher (S31), thereby genomically recombining the target cell with the objective substance (S32). Note that FIG. 3 illustrates an example including both the FFT-10 and the FFT-20 as a preferable example.

This can be performed, for example, by adding a substance delivery carrier in the form of a lipid composition to a target cell contained in a culture vessel or any reaction vessel. Warming at 37°C or higher can be performed, for example, by maintaining in an incubator or heating using a warming device such as another heater or a warming system. The warming conditions are as described above. For example, the warming at 37°C or higher may be at 40 to 42°C.

The objective substance may be a nucleic acid fragment such as RNA and/or DNA, a desired vector, a plasmid vector, a mixture with a protein, or the like. A substance that promotes genomic recombination of interest may be further included in the substance delivery carrier. In addition, if necessary, an additional substance may be added from the outside to the contact field between the target cell and the substance delivery carrier. Warming at 37°C or higher can be performed, for example, by maintaining in an incubator or heating using a warming device such as another heater or a warming system. In addition, after the contact between the delivery carrier and the target cell, culturing the cell under an appropriate environment may be further included.

### (Fourth Arrangement)

According to a fourth arrangement, there is provided a method for producing a target cell by delivering an objective substance to a material cell which is a target cell, the method including, as shown in FIG. 4, bringing a substance delivery carrier including lipid particles containing at least FFT-10 and/or FFT-20 and the objective substance encapsulated in the lipid particles into contact with the target cell in vitro and warming the carrier to 37°C or higher (S41), culturing the warmed cell (S42), and delivering the objective substance to the target cell to produce a target cell (goal cell) (S43). Note that FIG. 4 illustrates an example including both the FFT-10 and the FFT-20 as a preferable example.

For example, a substance delivery carrier in the form of a lipid composition is added to a target cell contained in a culture vessel or any reaction vessel. Warming at 37°C or higher can be performed, for example, by maintaining in an incubator or heating using a warming device such as another heater or a warming system.

The objective substance may be a nucleic acid fragment such as RNA and/or DNA, a desired vector, a plasmid vector, a mixture with a protein, or the like. A substance that promotes genomic recombination of interest may be further included in the substance delivery carrier. In addition, if necessary, an additional substance may be added from the outside to the contact field between the target cell and the substance delivery carrier. Warming at 37°C or higher can be performed, for example, by maintaining in an incubator or heating using a warming device such as another heater or a warming system. For example, the warming at 37°C or higher may be at 40 to 42°C. In addition, the method may further include culturing the target cell under an appropriate environment after the contact between the target cell and the delivery carrier.

By using this method, for example, it is possible to provide CAR-T cell production, iPS cell production, and the like. In such a case, the target cell is a cell intended to be produced, such as a CAR-T cell or an iPS cell. Each of such cell preparation methods can be performed with high efficiency.

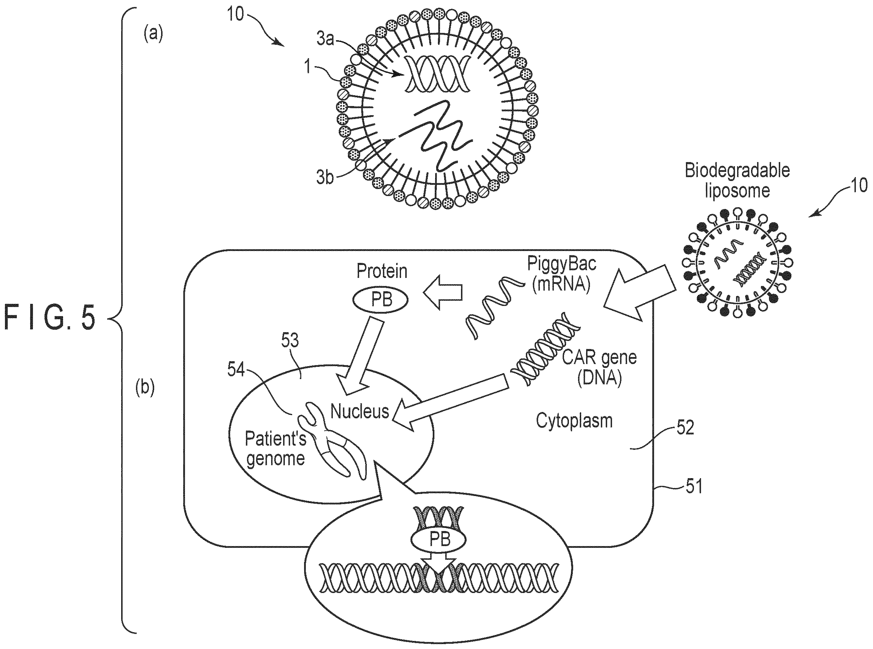

For example, in the case of producing a CAR-T cell, as shown in FIG. 5 (a), the substance of interest may be a nucleic acid 3a encoding any known CAR (chimeric antibody receptor) and a genome integration promoter 3b. In addition, the target cells may be used as a material fraction that is a material cell group containing material cells in a state of being contained in peripheral blood mononuclear cells (PBMC), a lymphocyte fraction, or the like. When CAR-T cells are used for treatment or diagnosis, they may be PBMCs collected from a subject to be treated, prevented and/or diagnosed. The adjustment of PBMCs from the peripheral blood may be performed by any method known per se, such as a density gradient centrifugation method such as ultracentrifugation using Ficoll.

The T cells contained in PBMCs can be cultured, for example, as follows. T cells contained in the separated PBMCs are cultured. The T cells are cultured using a commercially available T cell culture medium (TexMACS, Miltenyi biotec, AlyS705, Institute of Cell Sciences, and the like). Components that assist survival and proliferation of T cells, for example, artificial serum (artificially prepared serum containing no animal-derived component) and cytokines (such as interleukin 7, interleukin 15, etc.) may be added to the medium as necessary.

The nucleic acid 3a encoding the CAR is configured to be contained in the substance delivery carrier 10, delivered to the cytoplasm 52 of the target cell 51 to be a material, and sent from there to the nucleus 53 of the target cell 51, and then incorporated into the genome 54 of the cell. It may be configured to express the CAR on the cell membrane surface of the target cell by being incorporated into the genome 54 of the cell. The nucleic acid encoding the CAR is, for example, CAR-DNA, that is, double stranded DNA encoding the CAR gene sequence is preferred. CAR-DNA is configured to be taken up by the nucleus and inserted into the genome of the cell to express the CAR.

The genome integration promoter 3b may be, for example, a piggyBac (PB) gene such as PB-mRNA or the like. Such an enzyme gene is known as a transposase (DNA transferase) gene. By introducing the CAR gene and the transposase gene into a T cell which is a target cell, CAR-T (a cell in which the CAR gene is incorporated into the genome of the T cell) can be stably created. Representative transposases include piggyBac derived from moths, Sleeping Beauty derived from fish, and the like. Any of these may be used, but is not limited thereto. Furthermore, it is also possible to use a device that exhibits other genome integration promoting mechanisms. For example, the nucleic acid encoding the CAR may be incorporated into plasmid DNA and encapsulated in liposomes. Furthermore, the gene incorporated into the plasmid together with the PB gene may be enclosed as a gene set for CAR cell production. The PB gene may be DNA or mRNA.

In addition to the CAR-T cells, for example, even in the case of producing iPS cells, it is possible to use any arrangement in order to introduce a target gene into a target cell as an objective substance and efficiently cause genomic recombination at the time of cell dedifferentiation or silencing of various genes. In addition, the present invention is not limited thereto, and can be used for producing various cells by selecting an objective substance and a target cell.

### (Fifth Arrangement)

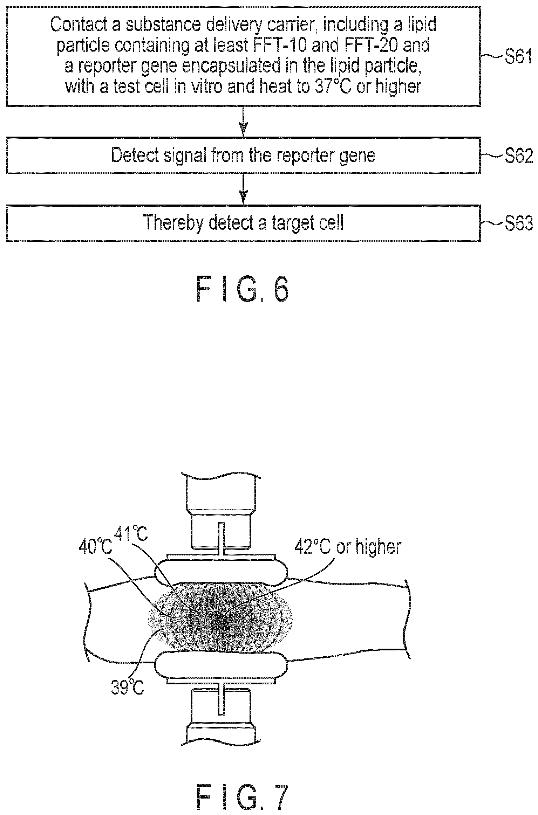

According to a fifth arrangement, a method for detecting a target cell is provided. In this method, as shown in FIG. 6, a substance delivery carrier including a lipid particle containing at least FFT-10 and/or FFT-20 and a reporter gene encapsulated in the lipid particle is brought into contact with a test cell in vitro and warmed to 37°C or higher (S61), a signal from the reporter gene is detected (S62), and thereby a target cell is detected (S63). Note that FIG. 6 illustrates an example including both the FFT-10 and the FFT-20 as a preferable example.

For example, a substance delivery carrier in the form of a lipid composition is added to a target cell contained in a culture vessel or any reaction vessel. Warming at 37°C or higher can be performed, for example, by maintaining in an incubator or heating using a warming device such as another heater or a warming system. The warming conditions are as described above. For example, the warming at 37°C or higher may be at 40 to 42°C.

The objective substance may be a reporter gene that generates a detectable signal in the cytoplasm or integrated genome of the introduced objective substance. Alternatively, a substance, such as an additional nucleic acid, may be included in the lipid particle linked to or in conjunction with such a reporter gene.

The reporter gene may be, for example, RNA containing mRNA of the reporter gene. The reporter gene is, for example, a gene encoding a reporter protein. The reporter protein is a protein that generates a detectable first signal. The reporter protein preferably has low cytotoxicity and is capable of detecting a signal in living cells.

The reporter protein is preferably selected from, for example, fluorescent proteins such as a green fluorescent protein, a red fluorescent protein, a blue fluorescent protein, and the like; luminescent enzyme proteins such as firefly luciferase, renilla luciferase, NanoLuc (registered trademark) luciferase, and the like; active oxygen generating enzymes such as xanthine oxidase, nitric oxide synthase, and the like; or chromogenic enzyme proteins such as β-galactosidase or chloramphenicol-acetyltransferase, and the like.

This RNA may further contain an additional sequence in addition to the mRNA of the reporter gene, may be modified so as to have degradation resistance, and may be encapsulated in the lipid particle in a state of being condensed by the nucleic acid condensing peptide.

The lipid particle may further comprise a gene activator that aids in expression of the reporter protein from the mRNA of the reporter gene and/or generation of the first signal from the reporter protein.

It may be a nucleic acid fragment such as RNA and/or DNA, a desired vector, a plasmid vector, a mixture with a protein, or the like. A substance that promotes genomic recombination of interest may be further included in the substance delivery carrier. In addition, if necessary, an additional substance may be added from the outside to the contact field between the target cell and the substance delivery carrier. Warming at 37°C or higher can be performed, for example, by maintaining in an incubator or heating using a warming device such as another heater or a warming system. For example, the warming at 37°C or higher may be at 40 to 42°C. The method may further include culturing the cell under an appropriate environment after warming at 37°C or higher.

Any of the arrangements described herein provides a technology capable of highly efficiently delivering a target molecule to a target cell. Such a technology can be carried out, for example, by bringing the lipid particles into contact with the target cells under a temperature environment within a certain range, for example, in a range of 40°C to 42°C, therefore, it is possible to achieve high efficiency without depending on the accuracy of the warming treatment.

### [EXAMPLES]

Hereinafter, examples of preparing and using the nucleic acid delivery carrier of the arrangement will be described.

### Experiment 1: Production of lipid particle encapsulating GFP gene

As the nucleic acid encapsulated in the lipid particle, mRNA of green fluorescent protein (GFP) gene was used.

FFT-10, FFT-20, DOPE, DOTAP, cholesterol, and DMG-PEG were dissolved in ethanol at a molar ratio shown in Table 1 below to obtain a lipid solution. While the lipid solution dispensed into the microtube was stirred with a vortex mixer, the nucleic acid was added dropwise and mixed to form particles. Each of the lipid particle solutions was 10 fold diluted with 10 mM HEPES (pH 7.3) and then concentrated with an ultrafiltration filter (Amicon Ultra 0.5 Ultracel -50 from Merck) to obtain nucleic acid delivery carriers of Examples 1 to 15.

**Table 1**

| | Molar ratio (FFT-10:FFT-20:DOPE:DOTAP: cholesterol:DMG-PEG) |
|---|---|
| Example 1 | 25:0:5:9:58:3 |
| Example 2 | 17:0:5:9:66:3 |
| Example 3 | 8:0:5:9:75:3 |
| Example 4 | 0:25:5:9:58:3 |
| Example 5 | 0:17:5:9:66:3 |
| Example 6 | 0:8:5:9:75:3 |
| Example 7 | 25:25:5:9:33:3 |
| Example 8 | 25:17:5:9:41:3 |
| Example 9 | 25:8:5:9:50:3 |
| Example 10 | 17:25:5:9:41:3 |
| Example 11 | 17:17:5:9:49:3 |
| Example 12 | 17:8:5:9:58:3 |
| Example 13 | 8:25:5:9:50:3 |
| Example 14 | 8:17:5:9:58:3 |
| Example 15 | 8:8:5:9:67:3 |

### Experiment 2 Measurement of gene expression intensity

A human breast cancer cell line (MCF7, manufactured by ATCC) cultured in MEM medium (manufactured by Thermo Fisher Scientific) and human mammary epithelial cells (HMEC, manufactured by Lifeline Cell Technology) cultured in MammaryLife BM medium (manufactured by Lifeline Cell Technology) were collected by centrifugation, and then seeded in a microtube at 4×10⁴ cells/tube. Each nucleic acid delivery carrier produced in Example 1 was added at 2 µL/tube. Each microtube was placed in a heat block and subjected to a warming treatment at 37 to 48°C for 0 to 17 minutes. Thereafter, the entire amount of the solution in the microtube was dispensed into a culture plate. The plate was placed in an incubator and the cells were cultured in an atmosphere of 37°C and 5% CO₂.

24 hours after the addition of the nucleic acid delivery carrier, the culture plate was removed from the incubator, and the fluorescence intensity of the GFP protein expressed from the GFP gene was measured with a microplate reader (infinite F200 Pro, manufactured by TECAN). The measurement was conducted according to the manual attached to the microplate reader.

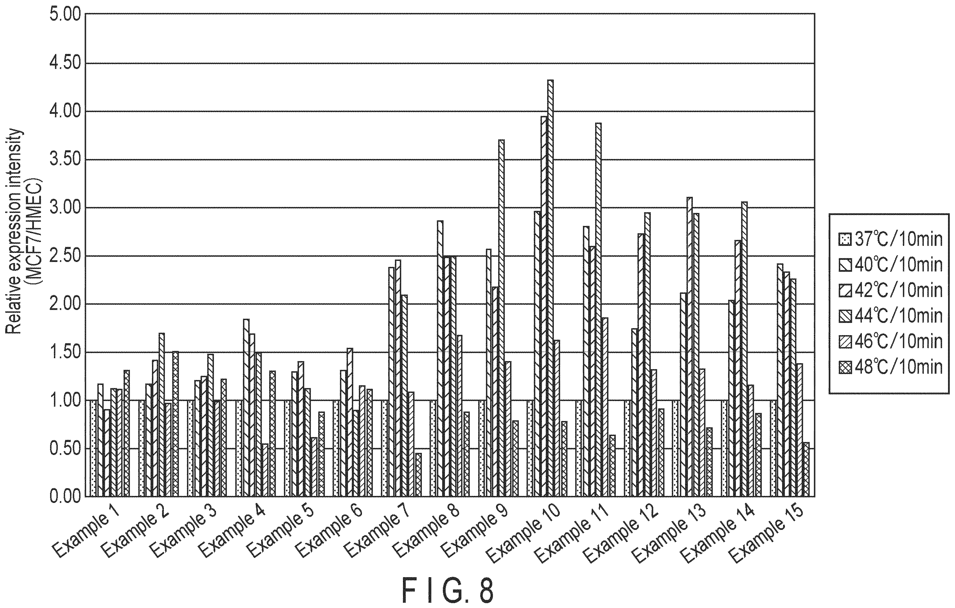

In FIG. 8, the results are shown, of measuring the fluorescence intensities of the GFP protein on the cells treated with worming at 37°C, 40°C, 42°C, 44°C, 46°C, or 48°C for 10 minutes, as relative fluorescence intensities obtained by dividing the fluorescence intensity of the MCF7 by the fluorescence intensity of the HMEC and setting the result of the warming at 37°C for 10 minutes to 1.

The relative fluorescence intensities were increased 1.2 to 1.8-fold on the cells treated with nucleic acid delivery carriers containing only one of FFT-10 and FFT-20 (Example 1 to 6) by the warming at 40 to 44°C. The relative fluorescence intensities increased 2 to 4 folds or more on the cells treated with nucleic acid delivery carriers containing both FFT-10 and FFT-20 (Example 7 to 15) by the warming at 40 to 44°C, and about 1.5-fold by the warming at 46°C. By the warming at 48°C, the relative fluorescence intensities were not significantly changed on the cells treated with any one of the nucleic acid delivery carriers examined.

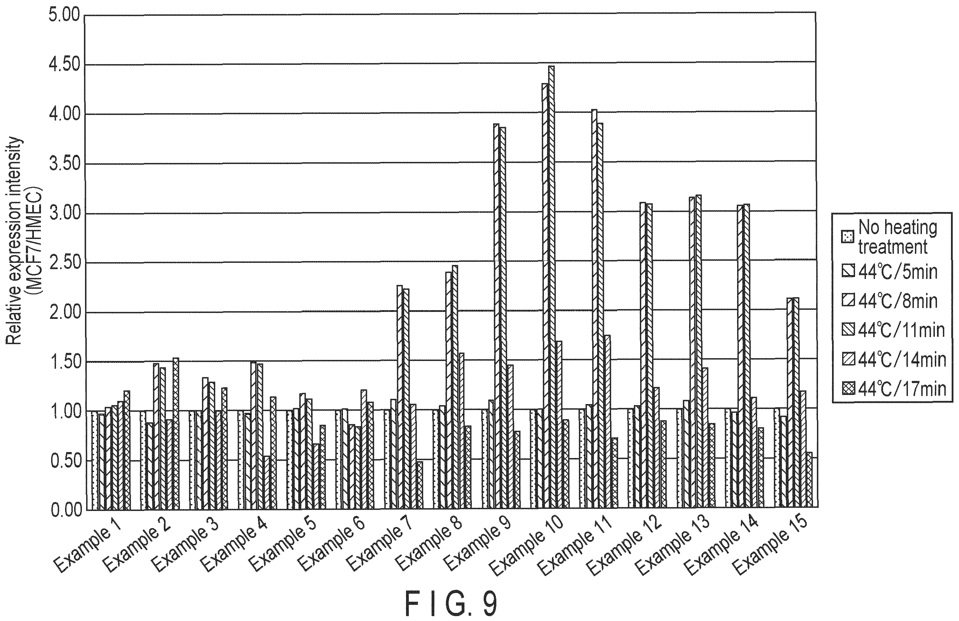

FIG. 9 shows the results of measuring the fluorescence intensity of GFP protein on the cells treated with warming at 44°C for 5 minutes, 8 minutes, 11 minutes, 14 minutes, and 17 minutes. Each data is represented by a relative fluorescence intensity obtained by dividing the fluorescence intensity of the MCF7 by the fluorescence intensity of the HMEC and setting the measurement result without the warming to 1. The relative fluorescence intensities were increased 1.2 to 1.5-fold on the cells treated with the nucleic acid delivery carriers containing only one of FFT-10 and FFT-20 (Example 1 to 6) by the warming for 8 to 14 minutes each. The relative fluorescence intensities was increased 1.5 to 4.5 folds or more on the cells treated with nucleic acid delivery carriers containing both FFT-10 and FFT-20 (Example 7 to 15) by the warming treatment for 8 to 14 minutes. The relative fluorescence intensities were not significantly changed on the cells treated with any one of nucleic acid delivery carriers by warming for 5 minutes and 17 minutes. From the above results, the improvement of cell directivity was shown on the nucleic acid delivery carrier containing both FFT-10 and FFT-20 by warming treatment under the conditions of a range of 40°C or more and less than 48°C for 8 minutes or more and less than 17 minutes.

### Experiment 3: Production of lipid particle encapsulating CAR gene and piggyBac gene

As the nucleic acid encapsulated in the lipid particle, plasmid DNA containing the CAR gene and m RNA of the piggyBac transposase gene were used.

FFT-10, FFT-20, DOPE, DOTAP, cholesterol and DMG-PEG were each dissolved in ethanol at a molar ratio of 15:30:4:9:38:4 to obtain a lipid solution. While the lipid solution dispensed into the microtube was stirred with a vortex mixer, the nucleic acid was added dropwise and mixed to form particles. Each of the lipid particle solutions was diluted 10-fold with 10 mM HEPES (pH 7.3) and then concentrated with an ultrafiltration filter (Amicon Ultra 0.5 Ultracel -50 from Merck) to obtain a nucleic acid delivery carrier of Example 16.

### Experiment 4 Evaluation of CAR-T cell production efficiency

Human peripheral blood mononuclear cells (PBMC, manufactured by Lonza) cultured in ALyS705 medium (manufactured by Institute of Cell Science) were collected by centrifugation, and then seeded into microtubes at 2 × 10⁵ cells/tube. The nucleic acid delivery carrier of Example 16 made in Example 3 was added at a nucleic acid amount of 2.5 µg/tube. The microtubes were placed in a heat block and subjected to no warming, warming at 42°C for 10 minutes, or warming at 42°C for 15 minutes. Thereafter, the entire amount of the solution in the microtubes was placed into each of culture plates, they were set in an incubator. The cells were cultured in an atmosphere of 37°C and 5% CO₂.

Two weeks after addition of the nucleic acid delivery carrier, the culture plates were removed from the incubator and each half of the culture fluid was collected in a microtube and washed with phosphate buffered saline (PBS) (from Thermo Fisher Scientific). The antibody-treated cells were washed with PBS, and then a biotin-labeled anti-human IgG (H + L) antibody (manufactured by Jackson IR) was added at 25-fold dilution, and the cells were incubated at 4°C for 30 minutes in a dark place. After washing with PBS, APC-labeled streptavidin (manufactured by BD) was added at 25-fold dilution, and the cells were incubated at 4°C for 30 minutes in a dark place. After washing the antibody-treated cells with PBS, the fluorescence intensity of APCs was measured with a flow cytometer (FACSVerse, manufactured by BD), and the expression rate of the CAR gene integrated into the genome of the cells by piggyBac was evaluated. The measurement was performed according to the manual attached to the flow cytometer.

Acute lymphocytic leukemia-derived CD 19 positive cells (NALM6, manufactured by ATCC) cultured in RPMI medium (manufactured by Thermo Fisher Scientific) were recovered by centrifugation, then mixed with PBMC included in a half remained potion of the culture solution described above, at a mixing ratio of PBMC:NALM 6 = 1:5, and seeded on a 96 well plate. The culture plate was placed in an incubator, and the cells were cultured at 37°C in a 5% CO₂ atmosphere. After 5 days from the co-culture, the culture plate was taken out from the incubator, and the entire amount of the culture solution was collected in a microtube and washed with PBS (manufactured by Thermo Fisher Scientific). After washing, an FITC-labeled anti-CD 19 antibody (manufactured by BD) was added at 25-fold dilution, and the mixture was incubated at 4°C for 30 minutes in a dark place. After washing the antibody-treated cells with PBS, the fluorescence intensity of FITC was measured with a flow cytometer (FACSVerse, manufactured by BD) to evaluate the NALM6 cell residual ratio, that is, the tumor cell killing performance of PBMCs expressing the CAR gene. The measurement was performed according to the manual attached to the flow cytometer.

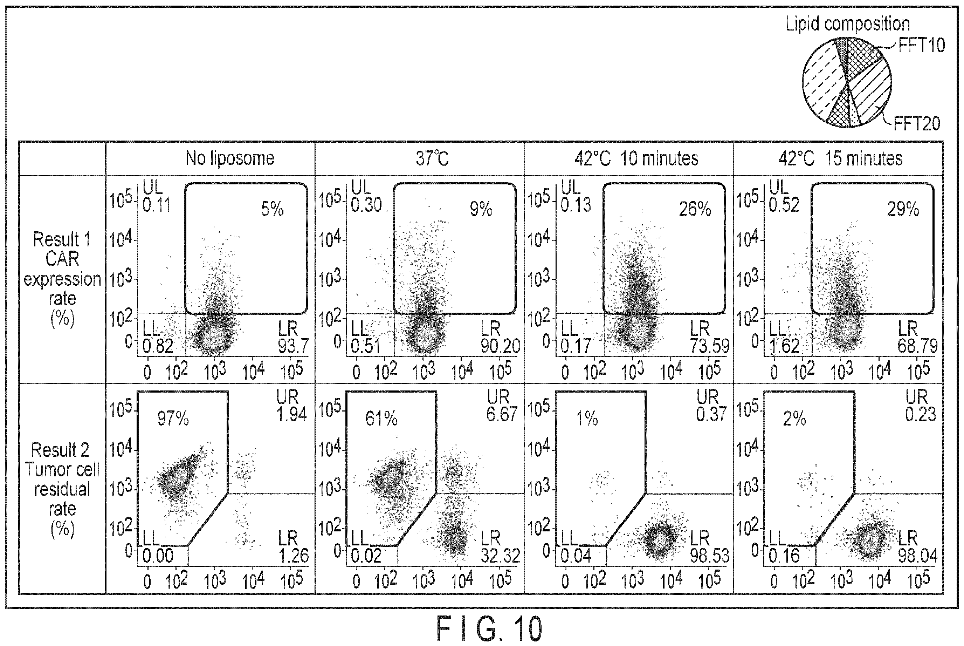

Table 2 shows the CAR gene expression rate and the tumor cell viability of each of; the PBMC cells without an addition of the nucleic acid delivery carrier; the PBMC cells with the addition of the nucleic acid delivery carrier of Example 16 but no warming treatment; the PBMC cells with the addition of the nucleic acid delivery carrier of Example 16 and the warming treatment at 42°C for 10 minutes; and the PBMC cells with the addition of the nucleic acid delivery carrier of Example 16 and the warming at 42°C for 15 minutes. In addition, an image obtained by the flow cytometer is shown in FIG. 10.

**Table 2**

| | CAR gene expression rate | Tumor cell viability |
|---|---|---|
| No nucleic acid delivery carrier | 5% | 97% |
| Example 16 + no warming treatment | 9% | 610 |
| Example 16 + warming treatment at 42°C for 10 minutes | 26% | 1% |
| Example 16 + warming treatment at 42°C for 15 minutes | 29% | 2% |

On the PBMC cells with the addition of the nucleic acid delivery carrier of Example 16 and warming at 42°C, the CAR gene expression rate was 25% or more and the tumor cell survival rate was 2% or less in both cases where the worming time was 10 minutes and 15 minutes. On the PBMC cells with the addition of the nucleic acid delivery carrier of Example 16 but no warming, a CAR gene expression rate of 9% and a tumor cell survival rate of 61%. Without nucleic acid delivery carriers, CAR gene expression was 5% and tumor cell viability was 97%. From the above results, the improvement was shown on the production efficiency of CAR-T cell generated with using the transposase piggyBac by conduction the warming treatment at 42°C for 10 to 15 minutes after addition the nucleic acid delivery carrier containing both FFT-10 and FFT-20. And the above results showed that CAR-T cells having high tumor cell killing performance could be produced.

From the above results, it was confirmed that a technology for highly efficiently delivering a target molecule to a target cell is provided by the lipid composition and each method according to the arrangement.

### (Sixth to Ninth Arrangements)

The second to fifth arrangements are methods performed in vitro. If desired, these methods can also be used as a treatment method, a diagnosis method, or the like under the control of a doctor. Furthermore, according to an arrangement, the following method is also provided.

### (Sixth Arrangement)

In such a case, a method for delivering an objective substance to a target cell is provided. In this method, for example, the term "in vitro" in the workflow illustrated in FIG. 2 is replaced with "in vivo or in tissue". In the method, a substance delivery carrier including a lipid particle containing at least FFT-10 and/or FFT-20 and an objective substance encapsulated in the lipid particle is brought into contact with a target cell in vivo or in tissue and warmed to 37°C or higher (S21), thereby delivering the objective substance to the target cell (S22).

### (Seventh Arrangement)

In addition, in such a method, the term "in vitro" in the workflow illustrated in FIG. 3 is replaced with "in vivo or in tissue". In the method, a substance delivery carrier including a lipid particle containing at least FFT-10 and/or FFT-20 and an objective substance encapsulated in the lipid particle is brought into contact with a target cell in vivo or in tissue and warmed to 37°C or higher (S31), thereby genomically recombining the target cell with the objective substance (S32).

### (Eighth Arrangement)

Further, such methods will replace the word "in vitro" in the workflow depicted in FIG. 4 with "in vivo or in tissue". The method includes bringing a substance delivery carrier containing an lipid particle containing at least FFT-10 and/or FFT-20 and an objective substance encapsulated in the lipid particle into contact with a material cell that is a target cell in vivo or in tissue and warming the substance delivery carrier to 37°C or higher (S41), culturing the warmed cell (S42), and delivering the objective substance to the material cell to produce a target cell (goal cell) (S43) .

### (Ninth Arrangement)

Further, in such methods, the term "in vitro" in the workflow illustrated in FIG. 6 is replaced with "in vivo or in tissue". The method includes contacting a substance delivery carrier including a lipid particle containing at least FFT-10 and/or FFT-20 and a reporter gene encapsulated in the lipid particle with a target cell in vivo or in tissue, and warming to 37°C or higher, to detect a signal from the reporter gene.

For the warming to 37°C or higher, for example, a high-frequency region warming mechanism (FIG. 7) employed in hyperthermia of cancer or the like may be used. Alternatively, heating may be performed using another heater, a warming device such as a bathtub, or a warming system. For example, the warming may be at 40°C to 42°C.

Delivery of the substance delivery carrier in vivo or in tissue is carried out by administering to the subject a composition comprising the substance delivery carrier. The administration route may include, not particularly limited, and for example, intravenous injection, subcutaneous injection, intramuscular injection, arterial injection, epidural injection, cerebrospinal space injection, intrathoracic injection, intraperitoneal injection, local/intralesional injection or the like, or systemic administration by infusion or the like. The administration schedule may be selected in consideration of the intended purpose, sex, age, body weight, or disease and conditions of the subject, and the like, and may be single administration, or may be continuous or periodic multiple administration. By administration, the substance delivery carrier is carried, for example, by blood to bring into contact with the target cells in the body. Lipid compositions and substance delivery carriers used in such methods are configured to meet the required grade and criteria as a suitable pharmaceutical composition to be administered to a living body.

According to these methods described above, an objective substance can be efficiently delivered to a target cell in a living body or a tissue, and detection, treatment, and/or diagnosis of the target cell can be performed.

Hereinafter, examples of arrangements performed in vivo or in tissue will be described.
(1) A method for delivering an objective substance, the method including: bringing a substance delivery carrier including a lipid particle containing at least FFT-10 and/or FFT-20 and an objective substance encapsulated in the lipid particle into contact with a target cell in vivo or in tissue; and warming the carrier to 37°C or higher, thereby delivering the objective substance to the target cell.
(2) A genome recombination method including: bringing a substance delivery carrier including a lipid particle containing at least FFT-10 and/or FFT-20 and an objective substance encapsulated in the lipid particle into contact with a target cell in vivo or in tissue and warming the carrier to 37°C or higher, thereby genomically recombining the target cell with the objective substance.
(3) A cell preparation method including: bringing a substance delivery carrier including a lipid particle containing at least FFT-10 and/or FFT-20 and an objective substance encapsulated in the lipid particle into contact with a material cell in vivo or in tissue and warming the carrier to 37°C or higher, culturing the warmed cell, and delivering the objective substance to the material cell, to prepare a target cell.
(4) A method for detecting a target cell, including: bringing a substance delivery carrier including a lipid particle containing at least FFT-10 and/or FFT-20 and a reporter gene encapsulated in the lipid particle into contact with a target cell in vivo or in tissue and warming the carrier to 37°C or higher, and detecting a signal from the reporter gene.

Although some arrangements of the present invention have been described, these arrangements have been presented as examples, and are not intended to limit the scope of the invention. These novel arrangements can be implemented in various other forms, and various omissions, substitutions, and changes can be made without departing from the gist of the invention. These arrangements and modifications thereof are included in the scope and gist of the invention, and are included in the invention described in the claims and the equivalent scope thereof.

While certain arrangements have been described, these arrangements have been presented by way of example only, and are not intended to limit the scope of the claims. Indeed, the compositions described herein may be embodied in a variety of other forms; furthermore, various omissions, substitutions and changes in the form of the compositions described herein may be made.

The arrangements as described above include clauses below.

### Clause 1

A lipid composition for delivering an objective substance, characterized by comprising
a substance delivery carrier containing a lipid particle and an objective substance encapsulated in the lipid particle, wherein
the lipid particle contains, as a constituent component thereof, at least a first lipid (FFT-10) of formula (I) and/or a second lipid (FFT-20) of formula (II) : 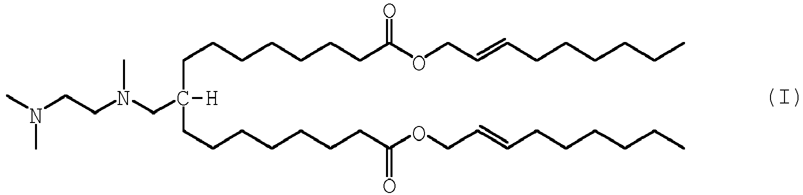 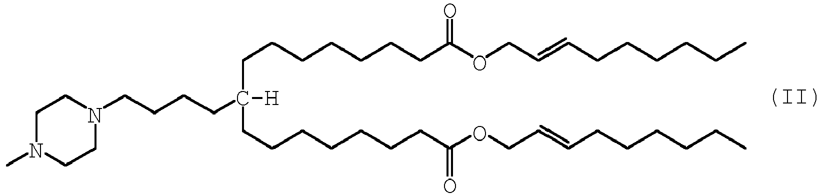 and the lipid composition is contacted with a target cell under an environment of 37°C or higher.

### Clause 2

The lipid composition of Clause 1, characterized in that the total content of the FFT-10 and the FFT-20 is 10% to 80% of the lipid particle.

### Clause 3

The lipid composition of Clause 1, characterized in that the contact environment is in a range of 40°C to 42°C.

### Clause 4

The lipid composition of Clause 1, characterized in that the contact environment is kept for 5 minutes to 20 minutes.

### Clause 5

The lipid composition of Clause 1, characterized in that the objective substance is a nucleic acid substance for genomic recombination.

### Clause 6

The lipid composition of Clause 1, characterized in that the nucleic acid substance is a nucleic acid fragment or a nucleic acid construct.

### Clause 7

A method for delivering an objective substance to a target cell, characterized by comprising
- contacting a substance delivery carrier containing
   a lipid particle containing at least a first lipid of formula (I) (FFT-10) and/or a second lipid of formula (II) (FFT-20): 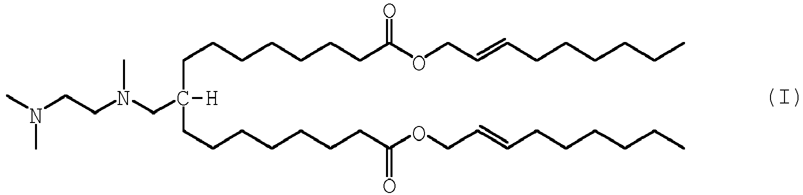 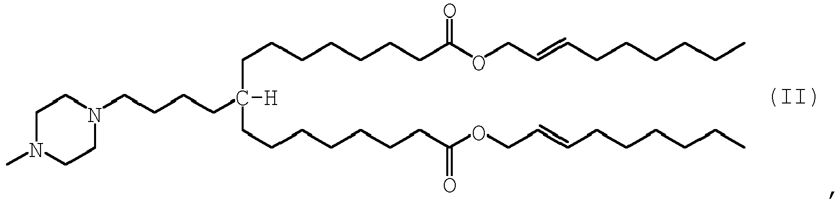 and
   an objective substance encapsulated in the lipid particle
   with a target cell in vitro; and
- warming the cell to 37°C or higher.

### Clause 8

The method of Clause 7, characterized in that the total content of the FFT-10 and the FFT-20 is 10% to 80% of the lipid particle.

### Clause 9

The method of Clause 7, characterized in that the warming temperature is in a range of 40°C to 42°C.

### Clause 10

A method for genomically recombining a target cell with an objective substance, characterized by comprising:
- contacting a substance delivery carrier containing
   a lipid particle containing at least a first lipid of formula (I) (FFT-10) and/or a second lipid of formula (II) (FFT-20): 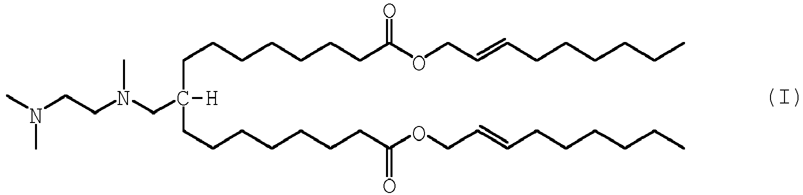 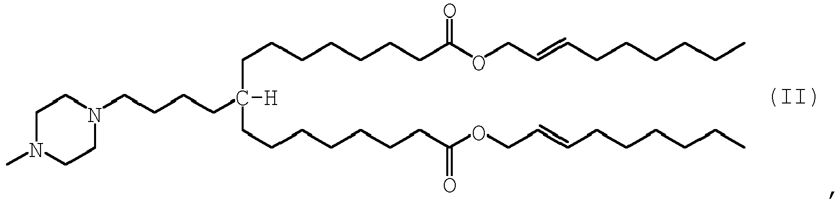 and
   an objective substance encapsulated in the lipid particle
   with a target cell in vitro; and
- warming the cell to 37°C or higher.

### Clause 11

The method of Clause 10, characterized in that the total content of the FFT-10 and the FFT-20 is 10% to 80% of the lipid particle.

### Clause 12

The method of Clause 10, characterized in that the warming temperature is in a range of 40°C to 42°C.

### Clause 13

The method of Clause 11, characterized by further comprising culturing the cell following the warming.

### Clause 14

A method for delivering an objective substance to a material cell to produce a target cell, characterized by comprising:
- contacting a substance delivery carrier containing
   a lipid particle containing at least a first lipid of formula (I) (FFT-10) and/or a second lipid of formula (II) (FFT-20): 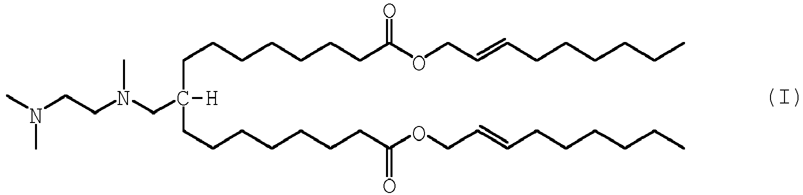 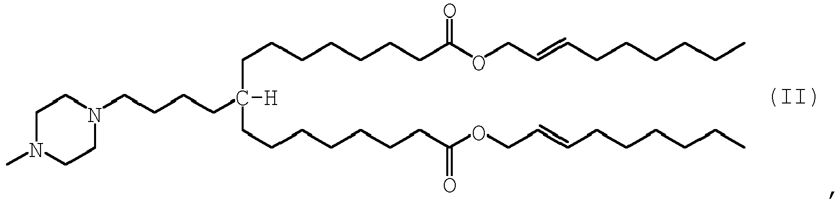 and
   an objective substance encapsulated in the lipid particle
   with a material cell in vitro;
- warming the cell to 37°C or higher; and
- culturing the warmed cell.

### Clause 15

The method of Clause 14, characterized in that the total content of the FFT-10 and the FFT-20 is 10% to 80% of the lipid particles.

### Clause 16

The method of Clause 14, characterized in that the warming temperature is in a range of 40°C to 42°C.

### Clause 17

A method for detecting a target cell, characterized by comprising:
- contacting a substance delivery carrier containing
   a lipid particle containing at least a first lipid of formula (I) (FFT-10) and/or a second lipid of formula (II) (FFT-20): 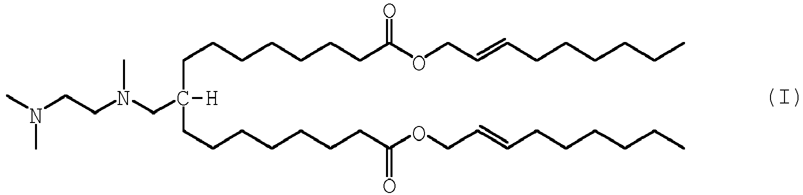 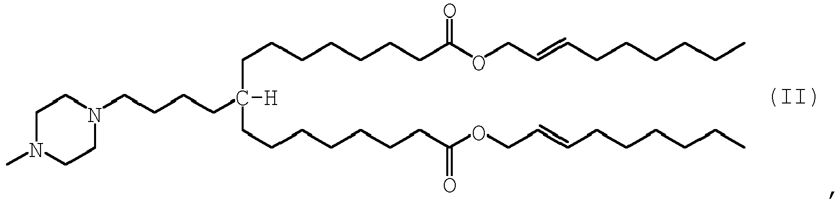 and
   a reporter gene encapsulated in the lipid particle
   with a target cell in vitro;
- warming the cells to 37°C or higher; and
- detecting a signal from the reporter gene.

### Clause 18

The method of Clause 17, characterized in that the total content of the FFT-10 and the FFT-20 is 10% to 80% of the lipid particles.

### Clause 19

The method of Clause 17, characterized in that the warming temperature is in a range of 40°C to 42°C.

### Clause 20

The method of Clause 17, characterized by further comprising culturing the target cell.

## Claims

1. A lipid composition for delivering an objective substance (3), **characterized by** comprising
a substance delivery carrier containing a lipid particle (1) and an objective substance (3) encapsulated in the lipid particle (1), wherein
the lipid particle (1) contains, as a constituent component thereof, at least a first lipid (FFT-10) of formula (I) 1a and/or a second lipid (FFT-20) of formula (II) 1b: 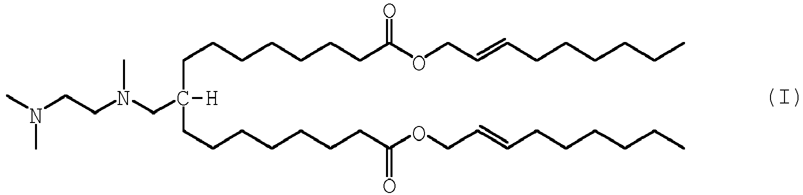 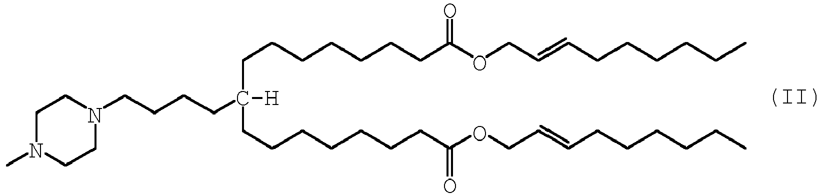 and the lipid composition is contacted with a target cell under an environment of 37°C or higher.

2. The lipid composition of claim 1, **characterized in that** the total content of the FFT-10 and the FFT-20 is 10% to 80% of the lipid particle (1).

3. The lipid composition of claim 1 or 2, **characterized in that** the contact environment is in a range of 40°C to 42°C.

4. The lipid composition of any of claims 1 to 3, **characterized in that** the contact environment is kept for 5 minutes to 20 minutes.

5. The lipid composition of any of claims 1 to 4, **characterized in that** the objective substance (3) is a nucleic acid substance for genomic recombination.

6. The lipid composition of any of claims 1 to 5, **characterized in that** the nucleic acid substance is a nucleic acid fragment or a nucleic acid construct.

7. A method for delivering an objective substance (3) to a target cell, **characterized by** comprising
- contacting a substance delivery carrier (10) containing
a lipid particle (1) containing at least a first lipid of formula (I) (FFT-10) 1a and/or a second lipid of formula (II) (FFT-20) 1b: 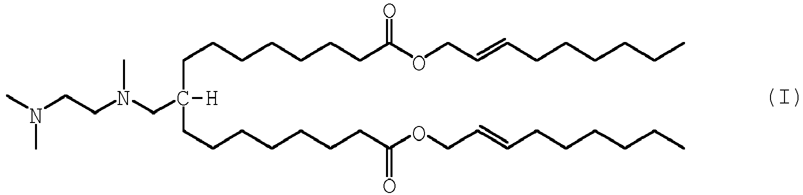 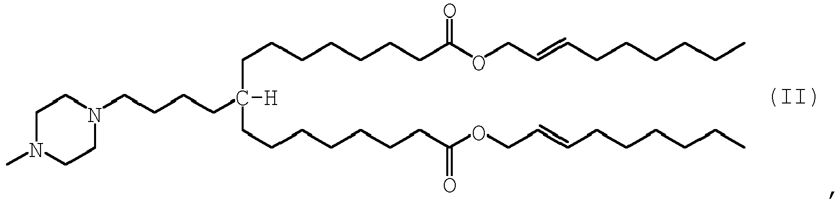 and
an objective substance (3) encapsulated in the lipid particle (1)
with a target cell in vitro; and
- warming the cell to 37°C or higher.

8. The method of claim 7, **characterized in that** the total content of the FFT-10 and the FFT-20 is 10% to 80% of the lipid particle (1).

9. The method of claim 7 or 8, **characterized in that** the warming temperature is in a range of 40°C to 42°C.

10. The method of any of claims 7 to 9, **characterized in that** the contacting between the lipid particle (1) and target cell leads to genomic recombination of the target cell.

11. The method of claim 10, **characterized in that** the total content of the FFT-10 and the FFT-20 is 10% to 80% of the lipid particle (1) and the warming temperature is in a range of 40°C to 42°C.

12. The method of any of claims 7 to 11, **characterized in that** the contacting between the lipid particle (1) and a target cell as material cell leads to production of another target cell.

13. The method of claim 12, **characterized in that** the total content of the FFT-10 and the FFT-20 is 10% to 80% of the lipid particle (1) and the warming temperature is in a range of 40°C to 42°C.

14. The method of claim 7, **characterized in that** the objective substance (3) encapsulated is a reporter gene, and
the method further comprises:
- warming the cells to 37°C or higher; and
- detecting a signal from the reporter gene to detect the target cell.

15. The method of claim 14, **characterized in that** the total content of the FFT-10 and the FFT-20 is 10% to 80% of the lipid particle (1) and the warming temperature is in a range of 40°C to 42°C.
